(19) Europäisches Patentamt
European Patent Office
Office européen des brevets

(11) Publication number: **0 050 964**
**B1**

(12) **EUROPEAN PATENT SPECIFICATION**

(45) Date of publication of patent specification: **04.03.87**

(21) Application number: **81304981.4**

(22) Date of filing: **22.10.81**

(51) Int. Cl.[4]: **C 07 D 407/06,** C 12 P 17/16, A 61 K 31/365, A 01 N 43/22 // C07H15/04, C07H13/04, C07H17/08 ,(C12P17/16, C12R1:465)

(54) **Macrocyclic lactones, their production and parasiticidal uses, compositions containing them, and a biological culture suitable for producing them.**

(30) Priority: **23.10.80 US 199900**

(43) Date of publication of application:
**05.05.82 Bulletin 82/18**

(45) Publication of the grant of the patent:
**04.03.87 Bulletin 87/10**

(84) Designated Contracting States:
**AT BE CH DE FR GB IT LI LU NL SE**

(56) References cited:
**US-A-4 196 280**

(73) Proprietor: **MERCK & CO. INC.**
**126, East Lincoln Avenue P.O. Box 2000**
**Rahway New Jersey 07065 (US)**

(72) Inventor: **Albers-Schonberg, George E.**
**30 Tyson Lane**
**Princeton New Jersey 08540 (US)**
Inventor: **Huang, Leeyuan**
**108 Summit Court**
**Westfield New Jersey 07090 (US)**
Inventor: **Hernandez, Sebastian**
**Virgen de Consolacion 8-2nd,**
**Madrid 27 (ES)**

(74) Representative: **Crampton, Keith John Allen et al**
**D YOUNG & CO 10 Staple Inn**
**London WC1V 7RD (GB)**

**EP 0 050 964 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with novel macrocyclic lactones, their production by the fermentation of a nutrient medium with a strain of the microorganism *Streptomyces Sp.* MA-5038.

In accordance with this invention, a class of substances is prepared by growing under controlled conditions a previously undescribed strain of microorganism. At least three distinct but closely related novel compounds are produced by *Streptomyces Sp.* MA-5038. The compounds are referred to herein as $A_1$, $A_2$ and $B_1$ and their postulated formulae are set forth below. Compound $A_1$ is the major component, produced in the greatest quantity. Compounds $A_2$ and $B_1$ are minor components of the isolated material. They are obtained by fermentation and recovered in substantially pure form as described herein.

Based on taxonomic studies, the microorganisms capable of producing these compounds are of a species of the genus *Streptomyces*. One such culture, isolated from soil, is designated MA-5038 in the culture collection of Merck & Co., Inc., Rahway, New Jersey. A sample of this culture, capable of producing the herein described compounds, was deposited, without restriction as to availability, in the permanent culture collection of the American Type Culture Collection at 12301 Parklawn Drive, Rockville, Maryland 20852, on 19 November 1979, and has been assigned the concession number ATCC 31587.

The morphological and cultural characteristics of *Streptomyces sp.* MA-5038 are set forth below:

CULTURAL CHARACTERISTICS OF:
*Streptomyces sp.* MA-5038 — ATCC 31587

(V = vegetative growth; A = aerial mycelium; SP = soluble pigment)

Morphology: Sporophores are produced at terminal portions of branching hyphae. Sporophores are in chains of more than 10 spores, forming loops, loose coils and loose spirals. Spores are spherical to oval or cylindrical, 0.9 μ × 1.2 μ (950X). Spore surface is smooth (electron microscopy).

Oatmeal agar (ISP Medium 3)
- V: Brown edged with yellow-tan
- A: Moderate, creamy white to pinkish cream (3ca)γ
- SP: None

Czapek Dox agar (sucrose nitrate agar)
- V: Tan
- A: Moderate, pale yellowish-pink (3ca)γ
- SP: Pink

Glycerol asparagine agar (ISP Medium 5)
- V: Cream to light tan
- A: Moderate, cream with pink cast
- SP: Pale yellowish-rose

Inorganic salts-starch agar (ISP Medium 4)
- V: Yellowish-tan
- A: Moderate, cream white
- SP: None

Yeast extract-dextrose + salts agar
- V: Brown edged with light brown and yellow tan segments
- A: Moderate, creamy white with pink cast
- SP: None

Yeast extract-malt extract agar (ISP Medium 2)
- V: Brown edged with light brown and yellow-tan segments
- A: Moderate, creamy white to pale yellowish pink (3ca)γ
- SP: None

Peptone-iron-yeast extract agar
- V: Tan
- A: None
- SP: None
- Melanin: Negative
- $H_2S$ production: Negative

Nutrient starch agar
- V: Tan
- A: Sparse, whitish
- SP: None
- Hydrolysis of starch moderate

Nutrient gelatin agar
- V: Tan
- A: Sparse, whitish
- SP: None
- Liquefaction of gelatin moderate

Nutrient tyrosine agar
V: Tan
A: Sparse, whitish
SP: Very slight browning of medium
Decomposition of tyrosine: Moderate Carbon utilization
Pridham-Gottlieb basal medium + 1% carbon source; + = growth; ± = growth poor or questionable; − = no growth as compared to negative control (no carbon source).
Glucose +
Arabinose +
Cellulose −
Fructose +
Inositol +
Lactose +
Maltose +
Mannitol +
Mannose +
Raffinose +
Rhamnose +
Sucrose +
Xylose +
Temperature range: (Yeast extract-dextrose + salts agar)
28°C — Good vegetative growth, moderate aerial growth and sporulation
37°C — Good vegetative growth, no aerial growth
50°C — No growth
Oxygen requirements (Stab culture in yeast extract-dextrose + salts agar):

Aerobic

All readings taken after three weeks at 28°C unless noted otherwise. pH of all media approximately neutral (6.8—7.2).

*Color number designations taken from Color Harmony Manual, 1958 4th Edition, Container Corporation of America, Chicago, Illinois.

A careful comparison of the foregoing data with published descriptions, including Bergey's Manual of Determinative Bacteriology (Eighth Edition) of known microorganisms reveals significant differences that the microorganism should be classified as a new species. On this basis, it was designated *Streptomyces sp.* MA-5038.

The above description is illustrative of a strain of *Streptomyces sp.* MA-5038 that can be used in the production of the present compounds. However, the present invention also embraces mutants of the above described microorganism. For example, those mutants which are obtained by natural selection or those produced by mutating agents including ionizing radiation such as ultraviolet irradiation, or chemical mutagens such as nitrosoguanidine are also included within the ambit of this invention.

The novel compounds are produced during the aerobic fermentation of suitable aqueous nutrient media under conditions described hereinafter, with a producing strain of *Streptomyces sp.* MA-5038. Aqueous media such as those used for the production of many antibiotic substances are suitable for use in this process for the production of these macrocyclic compounds.

Such nutrient media contain sources of carbon and nitrogen assimilable by the microorganism and generally low levels of inorganic salts. In addition, the fermentation media may contain traces of metals necessary for the growth of the microorganisms, and production of the desired compounds. These are usually present in sufficient concentrations in the complex sources of carbon and nitrogen, which may be used as nutrient sources, but can, of course, be added separately to the medium if desired.

In general, carbohydrates such as sugars, for example dextrose, sucrose, maltose, lactose, dextran, cerelose, corn meal and oat flour, and starches are suitable sources of assimilable carbon in the nutrient media. The exact quantity of the carbon source which is utilized in the medium will depend, in part, upon the other ingredients in the medium, but it is usually found that an amount of carbohydrate between 0.5 and 5% by weight of the medium is satisfactory. These carbon sources can be used individually or several such carbon sources may be combined in the same medium.

Various nitrogen sources such as yeast hydrolysates, yeast autolysates, yeast cells, tomato paste, corn meal, oat flour, soybean meal, casein hydrolysates, yeast extracts, corn steep liquors, distillers solubles, cottonseed meal and meat extract are readily assimilable by *Streptomyces sp.* MA-5038 in the production of the compounds. The various sources of nitrogen can be used alone or in combination in amounts ranging from 0.2 to 6% by weight of the medium.

Among the nutrient inorganic salts that can be incorporated in the culture media are the customary salts capable of yielding ions such as those of sodium, potassium, magnesium ammonium, calcium, phosphate, sulfate, chloride and carbonate. Also, included are trace metals such as cobalt and manganese.

It should be noted that the media described hereinbelow and in the Examples are merely illustrative and are not intended to be limitative.

The following are Examples of media suitable for growing strains of *Streptomyces sp.* MA-5038.

Medium A

| | |
|---|---|
| Dextrose | 1.0 g. |
| Soluble starch | 10.0 g. |
| Beef extract | 3.0 g. |
| Yeast autolysate | 5.0 g. |
| NZ Amine-E | 5.0 g. |
| $MgSO_4.7H_2O$ | 0.05 g. |
| $KH_2PO_4$ | 0.182 g. |
| $Na_2HPO_4$ | 0.190 g. |
| $CaCO_3$ | 0.5 g. |
| Distilled water | 1000 ml |
| pH 7.0—7.2 | |

Medium B

| | |
|---|---|
| Tomato paste | 20.0 g. |
| Primary yeast | 10.0 g. |
| Dextrin (CPC starch) | 20.0 g. |
| $CoCl_2.6H_2O$ | 0.005 g. |
| Distilled water | 1000 ml. |
| pH 7.2—7.4 | |

Medium C

| | |
|---|---|
| Corn meal | 20.0 g. |
| Distillers solubles | 10.0 g. |
| Soybean meal | 15.0 g. |
| Sodium citrate | 4.0 g. |
| $CaCl_22H_2O$ | 0.5 g. |
| $MgSO_4.7H_2O$ | 0.1 g. |
| $CoCl_2.6H_2O$ | 0.01 g. |
| $FeSO_4.2H_2O$ | 0.01 g. |
| Polyglycol P2000 (Polypropylene glycol mw 2000) | 2.5 ml. |
| Distilled water | 1000 ml. |
| pH 6.5 | |

Medium D

| | |
|---|---|
| Lactose | 20.0 g. |
| Distillers solubles | 15.0 g. |
| Autolyzed yeast (Ardamine pH) | 5.0 g. |
| Distilled water | q.s. to 1000 ml. |
| pH 7.0 | |

Medium E

| | |
|---|---|
| Tomato paste | 40.0 g. |
| Oat flour | 10.0 g. |
| Distilled water | 1000 ml. |
| pH 7.0 | |

The fermentation employing *Streptomyces sp.* MA-5038 can be conducted at temperatures ranging from 20°C to 40°C. For optimum results, it is most convenient to conduct these fermentations at a temperature in the range of 24°C to 30°C. Temperatures of 27—28°C are especially preferred. The pH of the nutrient medium suitable for producing the present compounds can vary from 5.0 to 9.0 with a preferred range of from 6.0 to 7.5.

Small scale fermentations are conveniently carried out by placing suitable quantities of nutrient medium in a flask employing known sterile techniques, inoculating the flask with either spores or vegetative cellular growth of *Streptomyces sp.* MA-5038, loosely stoppering the flask with cotton and permitting the fermentation to proceed in a constant room temperature of about 28°C on a rotary shaker for 3 to 10 days. For larger scale work, it is preferable to conduct the fermentation in suitable tanks provided

with an agitator and a means of aerating the fermentation medium. The nutrient medium is made up in the tank and after sterilization is inoculated with a suitable source of vegetative cellular growth of *Streptomyces sp.* MA-5038. The fermentation is allowed to continue for from 1 to 8 days while agitating and/or aerating the nutrient medium at a temperature in the range of from 24 to 37°C. The degree of aeration is dependent upon several factors such as the size of the fermentor and the agitation speed. Generally the larger scale fermentations are agitated at about 95 to 150 RPM and about 2 to 20 cubic feet per minute (CFM) of air, i.e. 0.95 to 9.5 litres/second of air.

The novel compounds of this invention are found primarily in the mycelium on termination of the *Streptomyces sp.* MA-5038 fermentation and may be removed and separated from one another as described below. The three major compounds are identified as $A_1$, $A_2$, and $B_1$. There are a considerable number of minor related components present in the broth which are at present unisolated and unidentified. The approximate ratio of the isolated major components is as follows:

$A_1$:$A_2$:$B_1$::10:1:4. Components $A_1$ and $A_2$ are acidic components whose Rf in thin-layer chromatography are dependent upon ammonia in the solvent system. The Rf of the $B_1$ component is not ammonia-dependent in its thin-layer chromatography analysis.

The separation of the novel compounds from the whole fermentation broth and the recovery of the individual compounds is carried out by solvent extraction and application of chromatographic fractionations with various chromatographic techniques and solvent systems.

The novel compounds have slight solubility in water, but are soluble in organic solvents. This property may be conveniently employed to recover the compounds from the fermentation broth. Thus, in one recovery method, the whole fermentation broth is combined with approximately an equal volume of an organic solvent. While any organic solvent may be used, it is preferable to use a water-miscible solvent such as acetone, methanol or ethanol. Generally several extractions are desirable to achieve maximum recovery. The solvent removes the compounds of the invention as well as other substances lacking the antiparasitic activity of those compounds. If the solvent is water-miscible, the solvent and water are combined into a single phase and filtered. The organic solvent is evaporated under reduced pressure and the remaining aqueous phase placed onto a chromatography column containing preferably, the highly cross-linked polystyrene resin, XAD-II (available from Rohm and Haas). The column retains the desired products and some impurities, but lets many of the impurities, particularly the water-soluble impurities, pass through. The column is washed with water to further remove impurities, and is then washed with an organic solvent, preferably acetone, methanol or ethanol. The solvent is evaporated and the residue further chromatographed, e.g. by column chromatography, thin-layer chromatography or preparative-layer chromatography, e.g. with silica gel, aluminium oxide, ion exchange resins or dextran gels as the chromatographic medium, with various solvents and combinations of solvents as the eluent. Thin-layer and preparative-layer chromatography may be employed to detect the presence of, and to isolate the individual compounds. The use of the foregoing and other known techniques will afford purified compositions containing mixtures of the novel compounds as well as the individual compounds themselves. The presence of the desired compounds is determined by analysing the various chromatographic fractions for biological activity or physico-chemical characteristics. The structures of the compounds have been determined by detailed analysis of the various spectral characteristics of the compounds, in particular their nuclear magnetic resonance, mass and ultraviolet and infrared spectra.

Based on these experimental data, the novel compounds are believed to have the following structural formula:

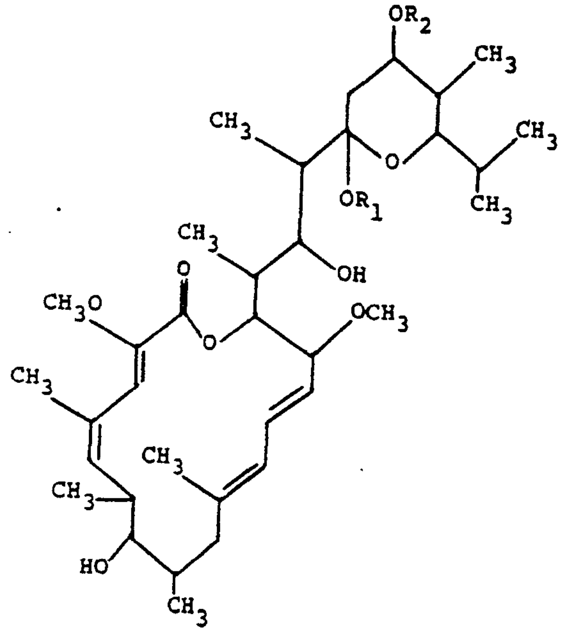

The variables $R_1$ and $R_2$ in the foregoing structure have the following definitions for the compounds of the invention.

| Compound | $R_1$ | $R_2$ |
|---|---|---|
| $A_1$ | H | —CO—CH=CH—COOH |
| $A_2$ | $—CH_3$ | —CO—CH=CH—COOH |
| $B_1$ | H | $CH_3$ |

The novel compounds of this invention have significant parasiticidal activity as insecticides and parasiticides. The compounds are of value against arthropods parasitizing man, biting insects and other dipterous pests causing annoyance to man.

The compounds are also active against household pests such as the cockroach, *Blatella sp.,* clothes moth, *Tineola sp.,* carpet bettle, *Attagenus sp.* and housefly, *Musca domestica.*

The compounds are also useful against insect pests of stored grains such as *Tribolium sp., Tenebrio sp.* and of agricultural plants such as spider mites, (*Tetranychus sp.*) aphids, (Acyrthiosiphon), migratory orthopterans such as locusts and immature stages of insects living on plant tissue.

The antiparasitic agents of this invention are also useful in the prevention and treatment of diseases caused by other parasites, for example, arthropod parasites such as ticks, lice, fleas, mites and other biting insects in domesticated animals and poultry. They are also effective in treatment of parasitic diseases that occur in other animals including humans. The optimum amount to be employed for best results will, of course, depend upon the particular compound, the species of animal to be treated and the type and severity of parasitic infection or infestation. Generally, good results are obtained with the novel compounds by the application to the insect of a solution or suspension of from about containing from 5 to 50 parts per million of the active compounds. The compound may be applied at one time or in divided doses over a relatively short period of time such as 1—5 days. With the preferred compounds of the invention, excellent control of such parasites is obtained in animals by administering a solution or suspension containing from 500 to 10,000 parts per million of the active compound in a single dose. Repeat treatments are given as required to combat reinfections and are dependent upon the species of parasite and the husbandry techniques involved. The techniques for administering these materials to animals are known to those skilled in the veterinary field.

The compounds of this invention show activity against the earthworm nematode *Caenorhabditis elegans* at *in vitro* levels of less than 30 micrograms per ml. The compounds also very effectively stimulate the release of γ-amino butyric acid from isolated rate brain synaptosomes.

When the compounds described herein are administered, compositions are provided in which the active compound or compounds are intimately dispersed in an inert carrier or diluent. By inert carrier is meant one that will not react with the insecticidal agent and that may be applied safely to the surface or area upon which the insect is found.

In using the compounds of this invention, the individual components may be isolated and purified and used in that form. Alternatively, mixtures of more than one of the individual components may be used. It is not necessary to completely separate the various compounds obtained from the purification of the fermentation broth. Generally, there is obtained a mixture containing two or more of the compounds, but having other unrelated compounds excluded therefrom, and such mixtures may be used for the prevention and treatment of insect infestations as described herein. Such a mixture generally will contain unequal proportions of the compounds. However, all of the compounds have substantial activity and the insecticidal activity of the mixture can be accurately determined.

When the compounds of this invention are used in combating agricultural pests that inflict damage upon crops while they are growing or while in storage, the compounds are applied using known techniques such as sprays, dusts and emulsions to the growing or stored crops to effect protection from such agricultural pests.

The compounds are particularly active against tapeworms, a form of cestode parasite, which infect man and animals. The tapeworm is a common parasite which, although often symptomless, can lead to various gastrointestinal and other disorders. Tapeworms such as *Hymenolepis diminuta, Hymenolepis nana, Taenia saginata, Taenia solium* and *Diphyllobothrium latam* are commonly found in such infections.

When the compounds of this invention are administered for the treatment of tapeworms, oral administration is preferred.

The active compounds of the present invention when used to treat the above disorders, are orally administered, for example, with an inert diluent an organic solvent, or with an assimilable edible carrier, or they may be enclosed in hard or soft gelatin capsules, or they may be compressed into tablets, or they may be incorporated directly with the food of the diet. For oral therapeutic administration, the active compound of this invention may be incorporated with excipients and used in such forms as tablets, troches, capsules, elixirs, suppositories, suspensions, syrups, wafers and chewing gum. The amount of active compound in such therapeutically useful compositions or preparations is such that a suitable dosage will be obtained.

The tablets, troches, pill, capsules and the like may also contain the following: a binder such as gum tragacanth, acacia, corn starch or gelatin; an excipient such as dicalcium phosphate; a disintegrating agent such as corn starch, potato starch or alginic acid; and a lubricant such as magnesium stearate; and a sweetening agent such as sucrose, lactose or saccharin or a flavoring agent such as peppermint, oil of wintergreen, or cherry flavoring may be added. When the dosage unit form is a capsule, it may contain, in addition to materials of the above type, a liquid carrier such as a fatty oil. Various other materials may be present as coatings or to otherwise modify the phsyical form of the dosage unit, for instance, tablets, pills or capsules may be coated with shellac, sugar or broth. A syrup or elixir may contain the active compounds, sucrose as a sweetening agent, methyl and propyl parabens as preservatives, a dye and a flavoring such as cherry or orange flavor. Of course, any material used in preparing any dosage unit form should be pharmaceutically pure and substantially non-toxic in the amount employed.

When used in the above formulation, the compound is employed at dosages sufficient to treat tapeworm infections. The dosages may be given a single daily dose or in divided dosages throughout the day. The specific dose given to a patient will vary with the severity of the condition, and the weight of the patient. As such, dosages of from 1 to 25 mg/kg/day have been found to be effective. Such doses may be divided to provide for up to 4 administrations per day. Preferred doses are of from 2 to 10 mg/kg/day. The treatment may be carried out over several days to ensure that the parasite is completely destroyed. Generally, treatment periods of from 1 to 21 days are adequate.

The following examples are being provided in order that the invention may be more fully understood. Such examples are not to be construed as being limitative of the invention.

Example 1

A frozen vial of *Streptomyces sp.* MA-5038 is used to innoculate 54 ml. of Medium A in a 250 ml. baffled Erlenmeyer flask. The flask is incubated at 28°C for 1 day at 220 r.p.m. on a rotary shaking machine. The flask is then stored at 8°C for 3 days before the contents are used to inoculate 44 ml. of Medium D using 5% inoculum in a −250 ml. unbaffled Erlenmeyer flasks. The flask is incubated for 4 days at 28°C at 220 r.p.m. on a rotary shaking machine.

Example 2

570 ml. of the culture broth as prepared in Example 1 is extracted with 600 ml. of acetone and filtering through a bed of diatomaceous earth filtering aid. The acetone is removed from the aqueous mixture by evaporation *in vacuo*. The aqueous suspension is passed through a water equilibrated 5.5 × 10 cm. column packed with XAD-II resin. The column is then washed with water and eluted with methanol. The methanol eluate is dried and evaporated affording 750 mg. of residue. 600 mg. of this residue is dissolved in 8 ml. of methanol and applied to 4 preparative layer chromatography plates coated with 500 μ of silica gel $GF_{254}$. The plates are developed with 15% methanol in chloroform. The progress of the development is followed by observing the ultraviolet light fluorescence quenching band. This band is removed and eluted with 15% methanol in chloroform. The eluate is dried and evaporated to dryness affording 171 ml. of residue which is a mixture of $A_1$, $A_2$ and $B_1$ components.

Example 3

900 ml. of fermentation broth as prepared in Example 1 is combined with 900 ml. of acetone and shaken and filtered through a bed of diatomaceous earth filtering aid. The filtrate is evaporated to remove the organic solvent and the aqueous residue is passed through a column of XAD-II resin in a 6 by 15.5 cm. column. The column is washed with water and eluted with 600 ml. of methanol. The methanol eluate is evaporated to dryness *in vacuo* and the residue dissolved in 10 ml. of methanol. This solution is placed on 6 preparative layer chromatography plates with a 2 mm. layer of silica $GF_{254}$. The plates are developed with chloroform containing 17% methanol. The plates are examined with an ultraviolet light and those bands where ultraviolet fluorescence is quenched are collected and eluted from the silica gel with 12% methanol in chloroform. The eluate is evaporated to dryness *in vacuo*. 121.8 mg. of the material removed from the preparative layer chromatography plates is dissolved in 2 ml. of methylene chloride and placed on 10 prewashed silical gel preparative layer chromatography plates with a 500 μ coating of silica gel $HF_{254}$. The plates are developed continuously for 20 hours in methylene chloride containing 10% methanol and 1% concentrated ammonium hydroxide. Non-acidic components, including $B_1$, of the mixture accumulate in the solvent front. The major components, $A_1$ having the lowest Rf and $A_2$ with a slightly higher Rf, completely separated under these conditions. The respective bands containing $A_1$ and $A_2$ are removed from the plates and eluted with chloroform containing 10% methanol. The eluates are filtered, evaporated, redissolved in methylene chloride, filtered, diluted with benzene and lyophilized affording 76.9 mg. of $A_1$ and 7.7 mg. of $A_2$.

Example 4

133 mg. of an enriched mixture of $A_1$, $A_2$ and $B_1$ compounds as prepared in Example 2 is dissolved in a minimum amount of methylene chloride and chromatographed on 4 prewashed plates with a 500 μ coating of silica gel $HF_{254}$, developing with methylene chloride containing 6% methanol and 1% concentrated ammonium hydroxide. This is followed by methylene chloride containing 10% methanol and 1%

concentrated ammonium hydroxide. The band containing the $A_1$ component is observed under ultraviolet light and removed from the plates, dissolved in methylene chloride containing 10% methanol followed by elution with methanol alone. The eluate is evaporated to dryness *in vacuo*, and the residue dissolved in methylene chloride, filtered, diluted with benzene and lyophilized affording 59 mg. of $A_1$.

Example 5

270 mg. of a mixture of $A_1$, $A_2$ and $B_1$ as prepared in Example 2 is dissolved in 3 ml. of methylene chloride and chromatographed on 9 prewashed preparative layer chromatography plates with a 250 µ coating of silica gel $HF_{254}$, and developed 3 times with chloroform containing 20% methanol and 2% concentrated ammonium hydroxide. Under these conditions components moving faster than $A_1$ and $A_2$ separated into several bands which are individually recovered. The major band of this mixture with the second highest Rf affords 41 mg., after separation from the plates and lyophilization as in Examples 3 and 4, of component $B_1$.

Example 6

Using nuclear magnetic resonance, mass spectroscopy and other analytical techniques, the structures of components $A_1$, $A_2$ and $B_1$, as elaborated above, have been determined. Some of the characteristic spectral data are set forth below.

$A_1$

Molecular Formula $C_{39}H_{60}O_{12}$

Molecular weight 720

Mass spectrometry: $M^+$ not observed characteristic fragment ions found at m/e 586.3861, $C_{35}H_{54}O_7$, calc. 586.3855 ($M^+$ — water and fumaric acid); 568.3809, $C_{35}H_{52}O_6$, calc. 568.3750 (586-water); 525.3176, $C_{32}H_{45}O_6$, calc. 525.3204 (568—$C_3H_7$)

Ultraviolet spectroscopy: λ max (methanol) 210 nm (ε 26,300), 245 nm (ε 35,590) and 284.5 nm (ε 15,880).

$A_2$

Molecular Formula $C_{40}H_{62}O_{12}$

Molecular weight 734

Mass spectrometry: $M^+$ observed m/e 734, characteristic fragment ions found at m/e 586 ($M^+$ — methanol and fumaric acid), 568 (586-water), 525 (568—$C_3H_7$)

Ultraviolet spectroscopy: λ max (methanol) 209 nm (ε 24,170), 246 nm (ε 32,870) and 283 nm (ε 14,120).

$B_1$

Molecular Formula $C_{36}H_{60}O_9$

Molecular weight 636

Mass spectrometry: $M^+$ not observed, characteristic fragmentations found at m/e 618 ($M^+$ — water), m/e 600 ($M^+$ — 2 water), m/e 586 ($M^+$ — water and methanol), m/e 568 (586-water) and m/e 525 (568—$C_3H_7$).

Ultraviolet spectroscopy: λ max (methanol) 246 nm (ε 38,200) and 284.5 nm (ε 16,950).

**Claims for the Contracting States: BE CH DE FR GB IT LI LU NL SE**

1. A compound having the formula

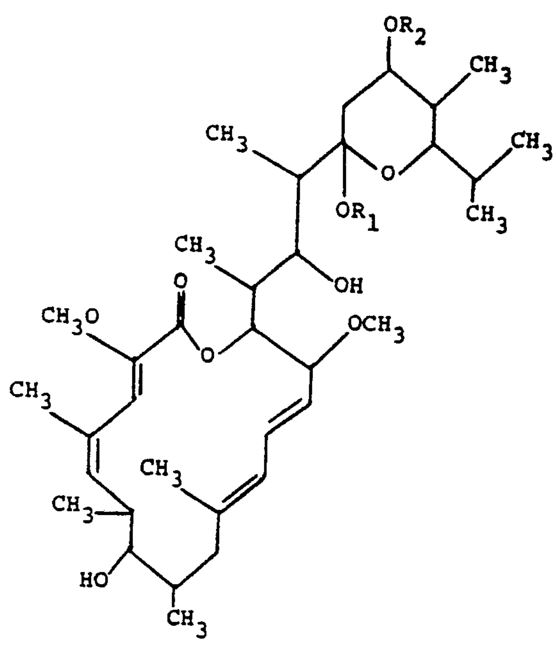

where the variables $R_1$ and $R_2$ have the following meanings for the compounds identified as $A_1$, $A_2$ and $B_1$:

| Compound | $R_1$ | $R_2$ |
|---|---|---|
| $A_1$ | H | —CO—CH=CH—COOH |
| $A_2$ | $—CH_3$ | —CO—CH=CH—COOH |
| $B_1$ | H | $—CH_3$ |

2. A process for the preparation of the compounds claimed in claim 1 which comprises fermenting with a producing strain of *Streptomyces sp.* ATCC 31587, an aqueous nutrient medium containing an assimilable source of carbon, an assimilable source of nitrogen, and inorganic salts under aerobic conditions and recovering said compounds from said fermentation broth.

3. A composition useful for the prevention and treatment of parasitic infections comprising an inert carrier in which one or more compounds as claimed in claim 1 is/are incorporated.

4. A compound as claimed in claim 1 for use in the prevention and treatment of parasitic infections by administration to an animal infected with parasites.

5. A composition useful for the prevention and treatment of agricultural pest infestations comprising an inert carrier in which one or more compounds as claimed in claim 1 is/are incorporated.

6. A method for the prevention and treatment of agricultural pest infestations that comprises applying to an area infested with such pests an effective amount of a compound as claimed in claim 1.

7. A composition useful for the prevention and treatment of tapeworm infection comprising an inert carrier in which one or more compounds as claimed in claim 1 is/are incorporated.

8. A compound as claimed in claim 1 for use in the prevention and treatment of tapeworms infections by administration to an animal infected with or liable to infection with tapeworms.

**Claims for the Contracting State: AT**

1. A process for the preparation of compounds having the formula:

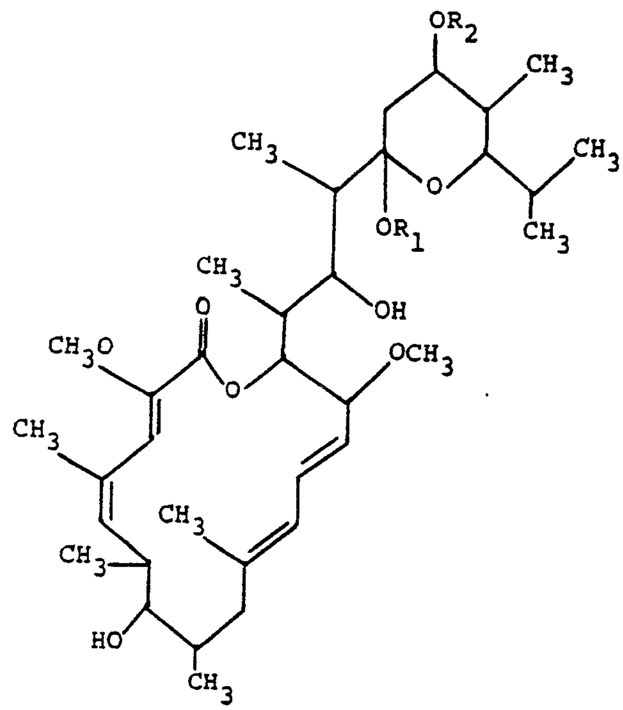

where the variables $R_1$ and $R_2$ have the following meanings for the compounds identified as $A_1$, $A_2$ and $B_1$:

| Compound | $R_1$ | $R_2$ |
|---|---|---|
| $A_1$ | H | —CO—CH=CH—COOH |
| $A_2$ | $—CH_3$ | —CO—CH=CH—COOH |
| $B_1$ | H | $—CH_3$ |

which comprises fermenting with a producing strain of *Streptomyces sp.* ATCC 31587, an aqueous nutrient medium containing an assimilable source of carbon, an assimilable source of nitrogen, and inorganic salts under aerobic conditions and recovering said compounds from said fermentation broth.

2. A composition useful for the prevention and treatment of parasitic infections comprising an inert carrier in which one or more compounds produced by a process as claimed in claim 1 is/are incorporated.

3. A compound produced by a process as claimed in claim 1 for use in the prevention and treatment of parasitic infections by administration to an animal infected with parasites.

4. A composition useful for the prevention and treatment of agricultrual pest infestations comprising an inert carrier in which one or more compounds produced by a process as claimed in claim 1 is/are incorporated.

5. A method for the prevention and treatment of agricultrual pest infestations that comprises applying to an area infested with such pests an effective amount of a compound produced by a process as claimed in claim 1.

6. A composition useful for the prevention and treatment of tapeworm infection comprising an inert carrier in which one or more compounds produced by a process as claimed in claim 1 is/are incorporated.

7. A compound produced by a process as claimed in claim 1 for use in the prevention and treatment of tapeworm infections by administration to an animal infected with or liable to infection with tapeworms.

**Patentansprüche für die Vertragsstaaten: BE CH DE FR GB IT LI LU NL SE**

1. Verbindung mit der Formel:

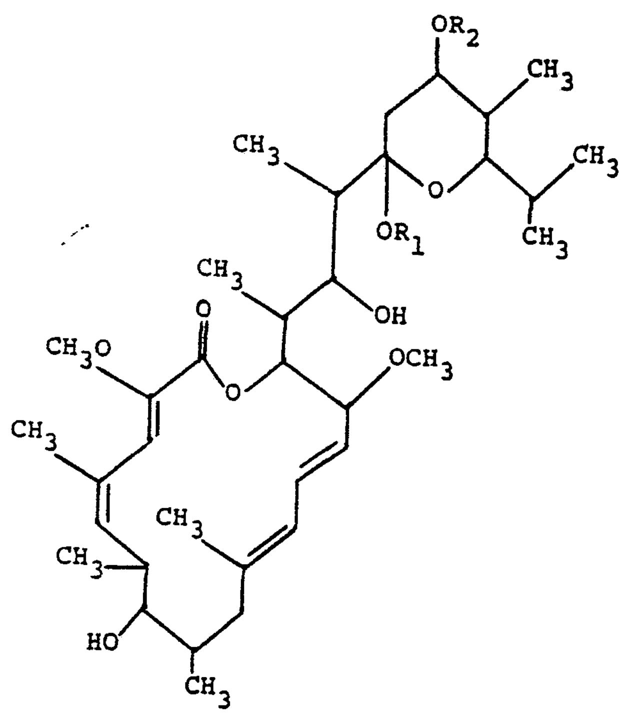

worin die Variablen $R_1$ und $R_2$ die nachstehende Bedeutung für die als $A_1$, $A_2$ und $B_1$ bezeichneten Verbindungen haben:

| Verbindung | $R_1$ | $R_2$ |
|---|---|---|
| $A_1$ | H | —CO—CH=CH—COOH |
| $A_2$ | $—CH_3$ | —CO—CH=CH—COOH |
| $B_1$ | H | $—CH_3$ |

2. Verfahren zur Herstellung der in Anspruch 1 beanspruchten Verbindungen, welches die Fermentierung unter aeroben Bedingungen eines eine assimilierbare Kohlenstoffquelle, eine assimilierbare Stickstoffquelle und anorganische Salze enthaltenden wässrigen Nährmediums mit einem produzierenden Stamm von *Streptomyces sp.* ATCC 31587 und die Gewinnung dieser Verbindungen aus der Fermentierungsbrühe umfasst.

3. Zur Vorbeugung und Behandlung von parasitären Infektionen verwendbare Zusammensetzung, enthaltend einen inerten Träger, in welchen eine oder mehrere in Anspruch 1 beanspruchte Verbindungen inkorporiert ist/sind.

4. Verbindung nach Anspruch 1 zur Verwendung bei der Vorbeugung und Behandlung von parasitären Infektionen durch Verabreichung an ein mit Parasiten infiziertes Tier.

5. Zur Vorbeugung und Behandlung von Schädlingsbefall in der Landwirtschaft verwendbare Zusammensetzung, enthaltend einen inerten Träger, in welchen eine oder mehrere in Anspruch 1 beansprüchte Verbindungen inkorporiert ist/sind.

6. Verfahren zur Vorbeugung und Behandlung von Schädlingsbefall in der Landwirtschaft, welches die Anwendung einer wirksamen Menge einer in Anspruch 1 beansprüchten Verbindung auf eine mit solchen Schädlingen befallene Fläche umfasst.

7. Zur Vorbeugung und Behandlung von Bandwurminfektionen verwendbare Zusammensetzung, enthaltend einen inerten Träger, in welchen einer oder mehrere in Anspruch 1 beanspruchte Verbindungen inkorporiert ist/sind.

8. Verbindung nach Anspruch 1 zur Vorbeugung und Behandlung von Bandwurminfektionen durch Verabreichung an ein mit Bandwürmern infiziertes oder der Gefahr einer Infektion damit unterliegendes Tier.

**Patentansprüche für den Vertragsstaat: AT**

1. Ein Verfahren zur Herstellung von Verbindungen der Formel:

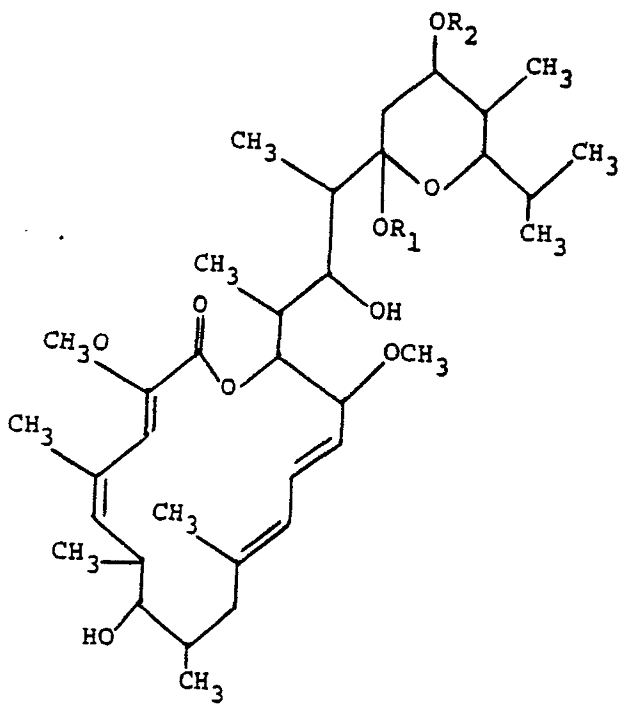

worin die Variablen $R_1$ und $R_2$ für die als $A_1$, $A_2$ und $B_1$ identifizierten Verbindungen die folgenden Bedeutungen haben:

| Verbindung | $R_1$ | $R_2$ |
|---|---|---|
| $A_1$ | H | —CO—CH=CH—COOH |
| $A_2$ | —$CH_3$ | —CO—CH=CH—COOH |
| $B_1$ | H | —$CH_3$ |

welches das Fermentieren eines wässerigen Nährmediums, das eine assimilierbare Kohlenstoffquelle, eine assimilierbare Stickstoffquelle und anorganische Salze enthält, mit einem Produktionsstamm von Streptomyces sp. ATCC 31587 unter aeroben Bedingungen, und das Gewinnen der genannten Verbindungen aus der genannten Fermentationsbrühe umfaßt.

2. Eine Zusammensetzung, die zur Verhinderung und Behandlung von Parasiteninfektionen nützlich ist, enthaltend einen inerten Träger, in welchem eine oder mehrere, nach eiinem wie in Anspruch 1 beansprüchten Verfahren erzeugte Verbindung(en) einverleibt ist bzw. sind.

3. Eine nach einem Verfahren, wie in Anspruch 1 beansprucht, erzeugte Verbindung zur Verwendung bei der Verhinderung und Behandlung von Parasiteninfektionen durch Verabreichung an ein durch Parasiten infiziertes Tier.

4. Eine Zusammensetzung, die zur Verhinderung und Behandlung von Befall durch Landwirtschaftsschädlinge nützlich ist, enthaltend einen inerten Träger, in welchem eine oder mehrere nach einem wie in Ansprüch 1 beanspruchten Verfahren erzeugte Verbindung(en) einverleibt ist bzw. sind.

5. Ein Verfahren zur Verhinderung und Behandlung von Befall durch Landwirtschaftsschädlinge, welches das Aufbringen einer wirksamen Menge einer Verbindung, die nach einem wie in Anspruch 1 beanspruchten Verfahren erzeugt ist, auf einen von solchen Schädlingen befallenen Bereich umfaßt.

6. Eine zur Verhinderung und Behandlung von Bandwurminfektion nützliche Zusammensetzung, enthaltend einen inerten Träger, in welchem eine oder mehrere, nach einem wie in Anspruch 1 beanspruchten Verfahren erzeugte Verbindung(en) einverleibt ist bzw. sind.

7. Eine nach einem wie in Anspruch 1 beanspruchten Verfahren erzeugte Verbindung zur Verwendung bei der Verhinderung und Behandlung von Bandwurminfektionen durch Verabreichung an ein Tier, das gefährdet ist, einer Infektion durch Bandwürmer zu unterliegen, oder das mit Bandwürmern infiziert ist.

**Revendications pour les Etats contractants: BE CH DE FR GB IT LI LU NL SE**

1. Composé ayant la formule:

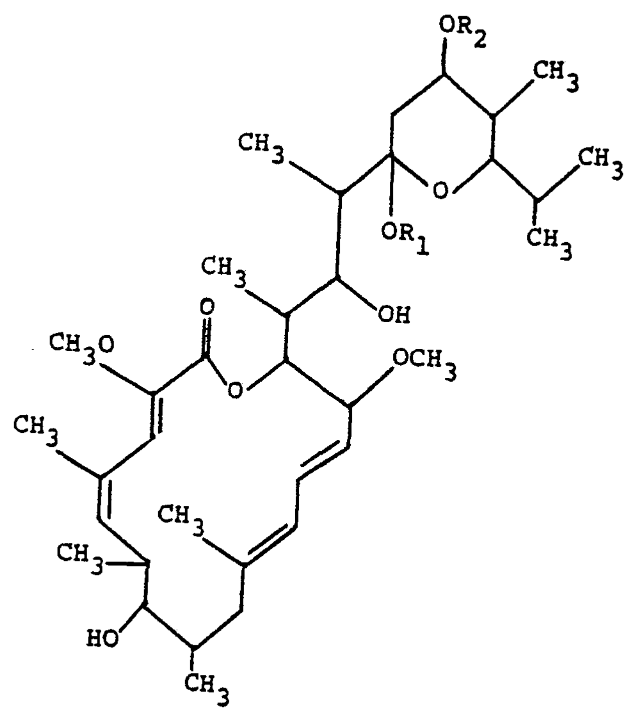

où les variables $R_1$ et $R_2$ ont les significations suivantes pour les composés identifiés comme $A_1$, $A_2$ et $B_1$:

| Composé | $R_1$ | $R_2$ |
|---|---|---|
| $A_1$ | H | —CO—CH=CH—COOH |
| $A_2$ | —$CH_3$ | —CO—CH=CH—COOH |
| $B_1$ | H | —$CH_3$ |

2. Procédé pour la préparation des composés revendiqués dans la revendication 1 qui comprend la fermentation avec une souche productrice de *Streptomyces sp.* ATCC 31587, un milieu nutritif aqueux contenant une source assimilable de carbone, une source assimilable d'azote et des sels minéraux dans des conditions aérobies et la récupération desdits composés à partir dudit bouillon de fermentation.

3. Composition utile pour la prévention et le traitement d'infections parasitaires comprenant un excipient inerte dans lequel un ou plusieurs composés tel(s) qu'il(s) est/sont revendiqué(s) dans la revendication 1 est/sont incorporé(s).

4. Composé tel qu'il est revendiqué dans la revendication 1 pour emploi dans la prévention et le traitement d'infections parasitaires par administration à un animal infecté par des parasites.

5. Composition utile pour la prévention et le traitement d'infestations par des parasites des cultures comprenant un excipient inerte dans lequel un ou plusieurs composés tel(s) qu'il(s) est/sont revendiqué(s) dans la revendication 1 est/sont incorporé(s).

6. Méthode pour la prévention et le traitement d'infestations par des parasites des cultures qui comprend l'application sur une surface infestée par de tels parasites d'une quantité efficace d'un composé tel qu'il est revendiqué dans la revendication 1.

7. Composition utile pour la prévention et le traitement d'infection par les ténias comprenant un excipient inerte dans lequel un ou plusieurs composés tel(s) qu'il(s) est/sont revendiqué(s) dans la revendication 1 est/sont incorporé(s).

8. Composé tel qu'il est revendiqué dans la revendication 1 pour emploi dans la prévention et le traitement d'infections par les ténias par administration à un animal infecté ou susceptible d'être infecté par des ténias.

**Revendications pour l'Etat contractant: AT**

1. Procédé pour la préparation de composés ayant la formule:

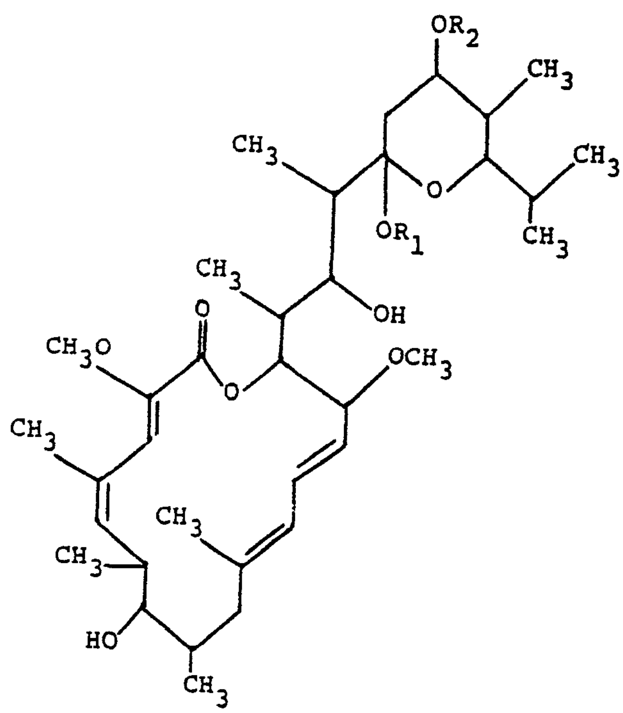

où les variables $R_1$ et $R_2$ ont les significations suivantes pour les composés identifiés comme $A_1$, $A_2$ et $B_1$:

| Composé | $R_1$ | $R_2$ |
|---|---|---|
| $A_1$ | H | —CO—CH=CH—COOH |
| $A_2$ | —$CH_3$ | —CO—CH=CH—COOH |
| $B_1$ | H | —$CH_3$ |

qui comprend la fermentation avec une souche productrice de *Streptomyces sp.* ATCC 31587, un milieu nutritif aqueux contenant une source assimilable de carbone, une source assimilable d'azote et des sels minéraux dans des conditions aérobies et la récupération desdits composés à partir dudit bouillon de fermentation.

2. Composition utile pour la prévention et le traitement d'infections parasitaires comprenant un excipient inerte dans lequel un ou plusieurs composés obtenus par un procédé tel qu'il est revendiqué dans la revendication 1 est/sont incorporé(s).

3. Composé obtenu par un procédé tel qu'il est revendiqué dans la revendication 1 pour emploi dans la prévention et le traitement d'infections parasitaires par administration à un animal infecté par des parasites.

4. Composition utile pour la prévention et le traitment d'infestations par des parasites des cultures comprenant un excipient inerte dans lequel un ou plusieurs composés obtenus par un procédé tel qu'il est revendiqué dans la revendication 1 est/sont incorporés.

5. Méthode pour la prévention et le traitement d'infestations par des parasites des cultures qui comprend l'application sur une surface infestée par de tels parasites d'une quantité efficace d'un composé obtenu par un procédé tel qu'il est revendiqué dans la revendication 1.

6. Composition utile pour la prévention et le traitment d'infection par les ténias, comprenant un excipient inerte dans lequel un ou plusieurs composés obtenu(s) par un procédé tel qu'il est revendiqué dans la revendication 1 est/sont incorporé(s).

7. Composé obtenu par un procédé tel qu'il est revendiqué dans la revendication 1 pour emploi dans la prévention et le traitement d'infections par les ténias par administration à un animal infecté ou susceptible d'être infecté par des ténias.